# FASCICULE DE BREVET EUROPEEN

(11) **EP 0 486 333 B1**
(45) Date de publication et mention de la délivrance du brevet: **14.12.1994**
(21) Numéro de dépôt: 91402668.7
(22) Date de dépôt: 07.10.1991
(51) Int. Cl.: C07C 19/08, C07C 17/20

(54) **Procédé de fabrication du tétrafluoro-1,1,1,2-éthane**
Verfahren zur Herstellung von 1,1,1,2-Tetrafluorethan
Process for the preparation of 1,1,1,2-tetra fluoroethane

(30) Priorité: 13.11.1990 FR 9014043
(43) Date de publication de la demande: 20.05.1992
(73) Titulaire: ELF ATOCHEM S.A., 92800 Puteaux (FR)
(72) Inventeur: Cheminal, Bernard, F-69530 Brignais (FR); Lacroix, Eric, F-69008 Lyon (FR); Lantz, André, F-69390 Vernaison (FR)

(56) Documents cités:
- EP-A- 0 331 991
- WO-A-90/08755
- FR-A- 2 014 711
- FR-A- 2 433 500
- US-A- 3 787 331
- US-A- 3 793 229
- US-A- 4 147 733

## Description

La présente invention concerne la fabrication du tétrafluoro-1,1,1,2 éthane par fluoration catalytique du chloro-1-trifluoro-2,2,2-éthane en phase gazeuse à l'aide d'acide fluorhydrique.

Le tétrafluoro-1,1,1,2 éthane (connu dans le métier sous la désignation F134a) est un composé potentiellement intéressant pour remplacer le dichlorodifluorométhane (F12) actuellement utilisé comme fluide frigorifique, mais suspecté de contribuer à l'affaiblissement de la couche d'ozone stratosphérique. On recherche donc actuellement des procédés économiques pour produire industriellement le F134a, l'un de ceux-ci étant la fluoration du chloro-1-trifluoro-2,2,2-éthane (connu dans le metier sous la désignation F133a).

La fluoration catalytique d'hydrocarbures chlorés ou bromés en phase gazeuse au moyen d'acide fluorhydrique est une méthode connue d'accès aux hydrocarbures fluorés.

Ainsi, dans le brevet US 2 744 147, on a décrit un catalyseur à base d'alumine promu par un métal (cobalt, nickel ou chrome) et son utilisation en lit fluidisé pour la fluoration d'haloalcanes à une température comprise entre 180 et 425°C.Ce brevet vise plus spécialement la fluoration des haloalcanes comportant 1 ou 2 atomes de carbone dont l'un au moins porte. au moins deux atomes d'halogène ayant un numéro atomique égal ou inférieur à 35, l'un au moins de ces atomes d'halogène étant le chlore ou le brome.

Il en est de même dans le brevet US 2 744 148 qui décrit. un catalyseur à base d'alumine promu par un métal (chrome, cobalt, nickel, cuivre ou palladium) et son emploi pour la fluoration d'haloalcanes en produits hautement fluorés. Ce brevet décrit aussi un procédé pour activer le catalyseur et convertir une partie de l'alumine en fluorures d'aluminium basiques.

Aucune indication n'est fournie dans ces brevets US en ce qui concerne la durée de fonctionnement dans le temps de ces formules catalytiques. De plus, aucun exemple ne décrit la fluoration du chloro-1-trifluoro-2,2,2-éthane, une fluoration qui présente la particularité d'être une réaction équilibrée conduisant à des conversions partielles de la matière première et dans laquelle, par ailleurs, la formation de composés insaturés engendre des phénomènes d'encrassement des catalyseurs par dépôt de coke, ce qui nuit à leur durée de vie.

Le brevet US 3 514 253 décrit des catalyseurs à base de fluorure d'aluminium imprégnés de sels de cuivre, cobalt, chrome ou nickel et leur utilisation dans la fluoration de composés aromatiques (trichlorobenzène) ou cycliques (octachlorocyclopentène).

Le procédé de fluoration décrit dans le brevet US 4 147 733 pour la fabrication du trifluoro-1,1,1 éthane ou du difluorométhane en présence de fluorures d'aluminium, chrome et/ou nickel, est caractérisé en ce que l'on additionne de la vapeur d'eau aux réactifs. Ce procédé ne s'applique pas au tétrafluoro-1,1,1,2 éthane dont le précurseur chloré (chloro-1 trifluoro-2,2,2 éthane) peut réagir avec l'eau dans les conditions opératoires décrites.

Le brevet FR 2 014 711 revendique un procédé de fluoration des haloalcanes, tout spécialement celle du trichloro1,1,2 trifluoro-1,2,2 éthane en dichloro-1,2 tétrafluoro1,1,2,2 éthane symétrique à l'aide d'un catalyseur constitué de fluorure d'aluminium et de faibles quantités de composés du fer, du chrome et éventuellement du nickel ; ce catalyseur convient également à la chlorofluoration de l'éthylène. Dans la première réaction, le catalyseur NiF₂/AlF₃ s'avère plus actif que le catalyseur mixte : (NiF₂+CrF₃)/AlF₃ (voir les essais n° 2 et 6 du tableau 1 de la page 9). Pour le même type de fluoration, le brevet US 3 793 229 décrit l'emploi de catalyseurs au zinc, chrome et nickel supporté sur AlF₃, et le brevet US 3 787 331 celui de catalyseurs au manganèse, chrome et éventuellement nickel supportés sur AlF₃. Aucun de ces trois brevets ne fait mention de la réaction de fluoration du chloro-1-trifluoro-2,2,2 éthane.

La demande de brevet WO 89/10341 revendique un procédé de fluoration de composés saturés ou insaturés en présence d'un catalyseur à base d'alumine très pure (contenant moins de 100 ppm de sodium, et poreuse) servant de support à des fluorures métalliques (nickel, cobalt, fer, manganèse, chrome, cuivre ou argent) Cette technique, qui exige un catalyseur de haute pureté, conduit en particulier à des sélectivités en tétrafluoro-1,1,1,2 éthane élevées mais souvent inférieures à 97,5 % et à une productivité souvent inférieure à 65 g/heure par litre de catalyseur.

Dans le brevet SU 466 202 est décrite une méthode de fluoration du chlorure de vinyle en présence d'un catalyseur constitué de fluorure d'aluminium, de fluorure de nickel et d'oxyde de chrome. Aucune mention n'est indiquée sur la préparation du catalyseur, sa durée de vie, ni surtout sur son activité dans la synthèse du tétrafluoro-1,1,1,2 éthane.

Les demandes de brevet EP 0 328 127 et 0 331 991 concernent spécifiquement la fabrication du tétrafluoro-1,1,1,2 éthane par fluoration catalytique du chloro-1-trifluoro-2,2,2 éthane en phase gazeuse à l'aide d'acide fluorhydrique. Selon le document EP 0 328 127 on opère en présence d'oxygène et utilise un catalyseur comprenant un métal choisi dans le groupe constitué par Co, Mn, Ni, Pd, Ag et Ru sur un support de fluorure d'aluminium. Dans le procédé du document EP 0 331 991 on utilise un catalyseur comprenant un métal des groupes VIII, VIIB, IIIB, IB ou un métal ayant un numéro atomique de 58 à 71, sur un support de fluorure d'aluminium ou de charbon. L'exemple 5 de ce dernier document montre qu'avec un catalyseur au nickel, à 350°C avec un temps de contact de 30 secondes et un rapport molaire HF/F133a de 10, le taux de conversion du F133a n'est que de 8 % et la sélectivité en F134a de 86,3 % seulement ; une augmentation de la température (400 et 425°C dans les exemples 6 et 7) améliore le taux de conversion du F133a, mais ne modifie pas sensiblement la sélectivité en F134a qui reste à environ 85 %.

La demande de brevet WO90/08755 décrit un procédé catalytique pour la préparation du tétrafluoro-1,1,1,2 éthane par réaction du trichloroéthylène avec HF dans un un réacteur unique alimenté en trichloroéthylène et en chloro-2 trifluoro-1,1,1 éthane recyclé. Le catalyseur comprend au moins un métal choisi parmi le chrome, les métaux des groupes VIII, VIIB, IIIB, IB et les métaux ayant un numéro atomique allant de 58 à 71, ledit métal étant de préférence déposé sur un support de fluorure d'aluminium ou de charbon.

Il a maintenant été trouvé qu'on peut obtenir une sélectivité en F134a très élevée (proche de 100 %) en utilisant un catalyseur mixte à base de nickel et de chrome.

L'invention a donc pour objet un procédé de fabrication du tétrafluoro-1,1,1,2 éthane (F134a) par fluoration catalytique du chloro-1 trifluoro-2,2,2 éthane (F133a) en phase gazeuse à l'aide d'acide fluorhydrique anhydre, caractérisé en ce que l'on utilise un catalyseur mixte composé d'oxydes, halogénures et/ou oxyhalogénures de nickel et de chrome déposés sur un support constitué de fluorure d'aluminium ou d'un mélange de fluorure d'aluminium et d'alumine.

Ce catalyseur peut être préparé de façon connue en soi à partir d'une alumine activée. Celle-ci peut dans une première étape être transformée en fluorure d'aluminium ou en mélange de fluorure d'aluminium et d'alumine, par fluoration à l'aide d'air et d'acide fluorhydrique, le taux de transformation de l'alumine en fluorure d'aluminium dépendant essentiellement de la température à laquelle est effectuée la fluoration de l'alumine (en général entre 200 et 450°C, de préférence entre 250 et 400°C). Le support est ensuite imprégné à l'aide de solutions aqueuses de sels de chrome et de nickel ou à l'aide de solutions aqueuses d'acide chromique, de sel de nickel et de méthanol (servant de réducteur au chrome).

Comme sels de chrome et de nickel, on préfère utiliser les chlorures, mais on peut aussi employer d'autres sels tels que, par exemple, les oxalates, formiates, acétates, nitrates et sulfates ou le bichromate de nickel pourvu que ces sels soient solubles dans la quantité d'eau susceptible d'être absorbée par le support.

Le catalyseur utilisé dans le procédé selon l'invention peut aussi être préparé par imprégnation directe de l'alumine activée à l'aide des solutions des composés de chrome et de nickel ci-dessus mentionnées. Dans ce cas, la transformation d'au moins une partie (70 % ou plus) de l'alumine en fluorure d'aluminium s'effectue lors de l'étape d'activation du catalyseur.

Les alumines activées à utiliser pour la préparation du catalyseur selon la présente invention sont des produits bien connus, disponibles dans le commerce Elles sont généralement préparées par calcination d'hydrates d'alumine à une température comprise entre 300 et 800°C. Les alumines activées, utilisables dans le cadre de la présente invention, peuvent contenir des teneurs importantes (jusqu'à 1000 ppm) de sodium sans que cela nuise aux performances catalytiques.

Le catalyseur selon l'invention peut contenir en poids de 0,5 à 20 % de chrome et de 0,5 à 20 % de nickel et, de préférence, entre 2 et 10 % de chacun des métaux dans un rapport atomique nickel/chrome compris entre 0,5 et 5, de préférence voisin de 1.

Avant de pouvoir catalyser la réaction de fluoration du F133a en F134a, le catalyseur selon l'invention doit être conditionné, c'est-à-dire transformé en constituants actifs et stables (aux conditions réactionnelles) par une opération préalable dite d'activation.

Ce traitement peut être réalisé soit "in situ" (dans le réacteur de fluoration) ou bien dans un appareillage adéquat conçu pour résister aux conditions d'activation. Celle-ci comprend généralement les étapes suivantes :
- séchage à basse température (100 à 150°C, de préférence 110 à 120°C) en présence d'air ou d'azote,
- séchage à haute température (350 à 450°C, de préférence 390 à 410°C) sous azote,
- fluoration à basse température (180 à 300°C, de préférence à 200°C environ) au moyen d'un mélange d'acide fluorhydrique et d'azote, en contrôlant la teneur en HF de façon que la température ne dépasse pas 300°C, et
- finition sous courant d'acide fluorhydrique pur à une température pouvant aller jusqu'à 450°C.

Pendant cette opération, les précurseurs catalytiques (halogénures de nickel et de chrome, chromate ou bichromate de nickel, oxyde de chrome) sont transformés en fluorures et/ou oxyfluorures correspondants, ce qui entraîne un dégagement d'eau et/ou d'acide chlorhydrique.

L'analyse chimique des éléments (chrome, nickel, fluor, aluminium, oxygène), après cette activation, permet de vérifier la composition minérale du catalyseur selon l'invention

Les conditions opératoires de synthèse du tétrafluoro1,1,1,2 éthane (F134a) par fluoration du chloro-1-trifluoro-2,2,2 éthane (F133a) en phase gazeuse à l'aide d'acide fluorhydrique en présence du catalyseur selon l'invention, sont les suivantes :
**a)** Le catalyseur peut fonctionner soit en lit fluidisé, soit en lit fixe. On préfère le second mode de fonctionnement car la réaction s'avère pratiquement athermique.
**b)** La température de réaction dépend de la pression de fonctionnement. A pression atmosphérique la température de réaction est comprise entre 300 et 375°C et, de préférence, comprise entre 330 et 360°C ; à plus haute pression (vers 15 bars absolus), la température optimale est comprise entre 350 et 420°C et, de préférence, comprise entre 375 et 410°C.
**c)** Le rapport molaire HF/F 133a peut varier entre 1 et 20, mais de préférence entre 2 et 5.
**d)** Le temps de contact, calculé comme le temps de passage des gaz (dans les conditions réactionnelles) à travers le volume de catalyseur en vrac, est compris entre 2 et 30 secondes. Il est de préférence compris entre 3 et 5 secondes sous pression atmosphérique et entre 5 à 25 secondes sous plus forte pression (vers 15 bars absolus).
**e)** L'addition d'oxygène pour maintenir l'activité catalytique n'est pas obligatoire et dépend des conditions de fonctionnement du catalyseur. Dans des conditions qui fournissent la productivité la plus élevée, et lorsque l'on désire une durée de vie compatible avec une application industrielle, la régénération continue ou discontinue du catalyseur peut être réalisée en présence d'oxygène ; sa concentration dans les gaz (réactifs ou inerte) doit alors être suffisante pour entraîner la combustion des produits carbonés responsables de la désactivation.
**f)** La pression de fonctionnement est comprise entre 1 et 20 bars absolus, de préférence comprise entre 12 et 16 bars absolus.

Les exemples suivants illustrent l'invention sans la limiter.

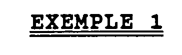

### 1A - PREPARATION ET ACTIVATION DU CATALYSEUR

Dans un évaporateur rotatif, on place 250 ml d'un support contenant en poids 73 % de fluorure d'aluminium et 27 % d'alumine, obtenu dans une étape précédente par fluoration d'alumine GRACE® HSA en lit fluidisé vers 300°C à l'aide d'air et d'acide fluorhydrique (concentration volumique de 5 à 10 % de cet acide dans l'air). L'alumine GRACE® HSA de départ présente les caractéristiques physicochimiques suivantes :
- forme : billes de 1-2 mm de diamètre
- surface BET : 223 m²/g
- volume poreux : 1,2 cm³/g [pour les rayons de pores compris entre 4 nm et 63 µm (40 Å et 63 microns)]
- teneur en sodium : 900 ppm
On prépare par ailleurs deux solutions aqueuses séparées :
**a)** solution chromique additionnée de chlorure de nickel contenant :

| | |
|---|---|
| - anhydride chromique | 12,5 g |
| - chlorure de nickel hexahydraté | 29 g |
| - eau | 40 g |

**b)** solution méthanolique contenant :

| | |
|---|---|
| - méthanol | 17,8 g |
| - eau | 50 g |

Le mélange de ces deux solutions est ensuite introduit, à température ambiante sous pression atmosphérique et en 45 minutes environ, sur le support en agitation. Le catalyseur est alors séché sous courant d'azote, en lit fluidisé, vers 110°C pendant 4 heures.

On charge 100 ml (72 g) de catalyseur sec dans un réacteur tubulaire en INCONEL® de diamètre intérieur 27 mm et l'on monte la température à 120°C sous courant d'azote, à pression atmosphérique. On maintient ce traitement pendant 15 heures puis on remplace l'azote par de l'air à la même température pendant 4 heures. La température est ensuite portée à 400°C sous courant d'azote, puis maintenue pendant 14 heures (durée de chauffage comprise).

La température est ensuite ramenée à 200°C sous courant d'azote pendant 15 heures et l'on remplace alors progressivement l'azote par de l'acide fluorhydrique en veillant à ce que l'augmentation de température n'excède pas 95°C.

On monte enfin la température à 450°C sous courant d'acide fluorhydrique pur (1 mole/heure) pendant 6 heures.

On redescend finalement à 350°C (sous courant d'azote) pour démarrer le test catalytique. Les propriétés physico-chimiques du catalyseur ainsi séché et activé sont les suivantes :

| - composition chimique (pondérale) | |
|---|---|
| . fluor | 61,6 % (≧ 95 % AlF₃) |
| . aluminium | 27,5 % |
| . nickel | 3,6 % |
| . chrome | 2,9 % |
| . oxygène | 4,4 % |

| - propriétés physiques : | |
|---|---|
| . surface BET : | 15,9 m²/g |
| . volume des pores d'un rayon compris entre 4 nm et 63 µm (40 Å et 63 microns) : | 0,430 cm³/g |
| . surface des pores de rayon supérieur à 4 nm (40 Å): | 16,0 m²/g |
| . surface des pores de rayon supérieur à 25 nm (250 Å): | 6,1 m²/g |
| . surface des pores de rayon compris entre 5 et 25 nm (50 et 250 Å) : | 6,1 m²/g |

### 1B - FLUORATION DU F133a EN F134a

Les performances du catalyseur ont été testées dans les conditions opératoires suivantes, sans addition d'oxygène :

| | |
|---|---|
| - volume de catalyseur (en vrac) | 75 ml |
| - température | 350°C |
| - pression | atmosphérique |
| - débit d'acide fluorhydrique | 1,09 moles/h |
| - débit de F133a | 0,26 moles/h |

c'est-à-dire un rapport molaire HF/F 133a = 4,2 ± 0,3 et un temps de contact de 3,9 ± 0,2 secondes dans les conditions réactionnelles.

Les gaz issus de la réaction sont débarrassés des hydracides par lavage à l'eau, puis séchés et analysés par C.P.V.

Le tableau 1 ci-après rassemble les principaux résultats obtenus au cours d'un test de 402 heures de fonctionnement continu sur cette même charge de catalyseur.

**TABLEAU 1**

| Durée cumulée de fonctionnement (heures) | **RESULTATS OBTENUS** | | |
|---|---|---|---|
| | Taux de conversion du F133a (%) | Sélectivité en F134a (%) | Productivité en F 134a (grammes/heure par litre de catalyseur) |
| 42 | 20,4 | 99,0 | 75 |
| 114 | 21,0 | 99,0 | 76 |
| 228 | 21,0 | 98,6 | 73 |
| 282 | 21,1 | 98,6 | 71 |
| 348 | 20,9 | 99,0 | 75 |
| 402 | 21,5 | 99,1 | 76 |

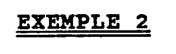

### 2A - PREPARATION ET ACTIVATION DU CATALYSEUR

On opère comme dans l'exemple 1A, mais en remplaçant l'anhydride chromique par le trichlorure de chrome hexahydraté et en supprimant le méthanol.

On prépare une seule solution aqueuse contenant :

| | |
|---|---|
| - trichlorure de chrome hexahydraté | 33,3 g |
| - chlorure de nickel hexahydraté | 29 g |
| - eau | 112 g |

et l'on imprègne 250 ml du même support avec cette solution, en une heure.

Le reste du traitement de séchage et d'activation est identique à celui décrit dans l'exemple 1A.

La composition chimique pondérale du catalyseur activé est la suivante :

| | |
|---|---|
| - fluor | 61,9 % |
| - aluminium | 27,9 % |
| - nickel | 3,6 % |
| - chrome | 3 % |
| - oxygène | 3,6 % |

Ses propriétés physiques sont les suivantes :

| | |
|---|---|
| . surface BET : | 36,1 m²/g |
| . volume des pores d'un rayon compris entre 4 nm et 63 µm (40 Å et 63 microns) : | 0,441 cm³/g |
| . surface des pores de rayon supérieur à 4 nm (40 Å): | 35,5 m²/g |
| . surface des pores de rayon supérieur à 25 nm (250 Å): | 4,3 m³/g |
| . surface des pores de rayon compris entre 5 et 25 nm (50 et 250 Å) : | 20,1 m²/g |

### 2B - FLUORATION DU F133a EN F134a

Ce catalyseur a été testé dans les mêmes conditions opératoires que celles décrites dans l'exemple 1B. Les résultats obtenus au cours d'un essai de 123 heures en fonctionnement continu, sans addition d'oxygène, sont rassemblés dans le tableau 2 ci-après.

**TABLEAU 2**

| Durée cumulée de fonctionnement (heures) | **RESULTATS OBTENUS** | | |
|---|---|---|---|
| | Taux de conversion du F 133a (%) | Sélectivité en F 134a (%) | Productivité en F 134a (grammes/heure par litre de catalyseur) |
| 23 | 21,2 | 98,6 | 73 |
| 41 | 20,8 | 98,6 | 75 |
| 58 | 20,9 | 98,6 | 75 |
| 123 | 20,4 | 98,5 | 73 |

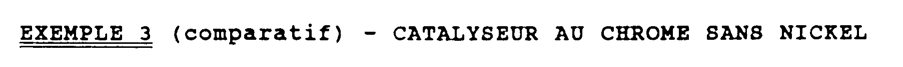

Le catalyseur a été préparé et activé en opérant exactement comme décrit dans l'exemple 1A, mais en supprimant les 29 g de chlorure de nickel hexahydraté dans la solution aqueuse a).

La composition chimique du catalyseur activé est la sutvante :

| | |
|---|---|
| - fluor | 63,3 % |
| - aluminium | 30,1 % |
| - chrome | 3,5 % |
| - oxygène | 3,1 % |

Ses propriétés physiques sont les suivantes :

| | |
|---|---|
| . surface BET : | 18,6 m²/g |
| . volume des pores d'un rayon compris entre 4 nm et 63 µm (40 Å et 63 microns) : | 0,438 cm³/g |
| . surface des pores de rayon supérieur à 4 nm (40 Å): | 21,3 m²/g |
| . surface des pores de rayon supérieur à 25 nm (250 Å): | 5,9 m²/g |
| . surface des pores de rayon compris entre 5 et 25 nm (50 et 250 Å) : | 9,8 m²/g |

Ce catalyseur a été testé dans les mêmes conditions opératoires que celles décrites dans l'exemple 1B. Les résultats obtenus au cours d'un essai de 126 heures en marche continue, sans addition d'oxygène, sont rassemblés dans le tableau 3 ci-après.

**TABLEAU 3**

| Durée cumulée de fonctionnement (heures) | **RESULTATS OBTENUS** | | |
|---|---|---|---|
| | Taux de conversion du F 133a (%) | Sélectivité en F 134a (%) | Productivité en F 134a (grammes/heure par litre de catalyseur) |
| 24 | 20,4 | 98,0 | 72 |
| 48 | 18,1 | 98,3 | 64 |
| 73 | 17,9 | 98,9 | 63 |
| 98 | 16,9 | 98,8 | 60 |
| 126 | 15,5 | 98,1 | 54 |

La comparaison de ces résultats avec ceux des tableaux 1 et 2 permet de conclure que l'association nickel-chrome selon l'invention est plus active et surtout plus stable dans le temps.

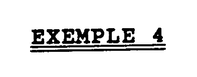

### 4A - PREPARATION ET ACTIVATION DU CATALYSEUR

On procède comme dans l'exemple 1A (même support) en modifiant comme suit les proportions des deux solutions aqueuses.
**a)** solution chromique de chlorure de nickel contenant :

| | |
|---|---|
| - anhydride chromique | 25 g |
| - chlorure de nickel hexahydraté | 58 g |
| - eau | 40 g |

**b)** solution méthanolique contenant :

| | |
|---|---|
| - méthanol | 17,8 g |
| - eau | 30 g |

Le séchage et l'activation du catalyseur sont ensuite strictement conduits comme indiqué dans l'exemple 1A.

Les propriétés physico-chimiques du catalyseur ainsi obtenu sont les suivantes :

| - composition chimique (pondérale) | |
|---|---|
| . fluor | 58,4 % |
| . aluminium | 24,6 % |
| . nickel | 6,8 % |
| . chrome | 5,1 % |
| . oxygène | 5,1 % |

| - propriétés physiques | |
|---|---|
| . surface BET | 15,1 m²/g |
| . volume des pores d'un rayon compris entre 4 nm et 63 µm (40 Å et 63 microns) | 0,382 cm³/g |
| . surface des pores de rayon supérieur à 4 nm (40 Å) | 20 m²/g |
| . surface des pores de rayon supérieur à 25 nm (250 Å) | 7,7 m²/g |
| . surface des pores de rayon compris entre 5 et 25 nm (50 et 250 Å) | 8,2 m²/g |

### 4B - FLUORATION DU F133a EN F134a

Les performances du catalyseur ont été testées dans les conditions opératoires suivantes :

| | |
|---|---|
| - volume de catalyseur (en vrac) | 175 ml |
| - température | 350°C |
| - pression (absolue) | 12 bars |
| - débit d'acide fluorhydrique | 5,35 moles/h |
| - débit de F133a | 2,67 moles/h |

Un rapport molaire HF/F133a compris entre 2 et 2,4, et un temps de contact compris entre 16,5 et 17,9 secondes, ont été maintenus au cours de cet essai.

Les gaz issus de la réaction sont détendus à pression atmosphérique, débarrassés des hydracides par lavage à l'eau, puis séchés et analysés par CPV.

Le tableau 4 ci-après rassemble les principaux résultats obtenus au cours d'un test de 54 heures de fonctionnement continu sur cette même charge de catalyseur.

**TABLEAU 4**

| Durée cumulée de fonctionnement (heures) | **RESULTATS OBTENUS** | | |
|---|---|---|---|
| | Taux de conversion du F 133a (%) | Sélectivité en F 134a (%) | Productivité en F 134a (grammes/heure par litre de catalyseur) |
| 12,5 | 13,8 | 97,3 | 205 |
| 29,5 | 13,4 | 97,4 | 210 |
| 36,5 | 13,3 | 97,2 | 206 |
| 53,5 | 13,3 | 97,4 | 210 |

## Revendications

1. Procédé de fabrication du tétrafluoro-1,1,1,2 éthane (F134a) par fluoration catalytique du chloro-1-trifluoro-2,2,2 éthane (F133a) à l'aide d'acide fluorhydrique gazeux anhydre, caractérisé en ce que l'on utilise un catalyseur mixte composé d'oxydes, halogénures et/ou oxyhalogénures de nickel et de chrome déposés sur un support constitué de fluorure d'aluminium ou d'un mélange de fluorure d'aluminium et d'alumine.

2. Procédé selon la revendication 1, caractérisé en ce que la teneur pondérale du catalyseur en nickel et en chrome est comprise entre 0,5 et 20 % pour chaque métal, le rapport atomique nickel/chrome étant compris entre 0,5 et 5, de préférence voisin de 1.

3. Procédé selon la revendication 2, caractérisé en ce que la teneur est comprise entre 2 et 10 % pour chaque métal.

4. Procédé selon l'une des revendications 1 à 3, caractérisé en ce que le support peut contenir jusqu'à 1000 ppm de sodium.

5. Procédé selon l'une des revendications 1 à 4, caractérisé en ce que la température de réaction est comprise entre 300 et 420°C, de préférence entre 330 et 410°C.

6. Procédé selon l'une des revendications 1 à 5, caractérisê en ce que la pression de fonctionnement est comprise entre 1 et 20 bars, de préférence entre 12 et 16 bars absolus.

7. Procédé selon l'une des revendications 1 à 6, caractérisé en ce que le rapport molaire HF/F 133a est compris entre 1 et 20, de préférence entre 2 et 5.

8. Procédé selon l'une des revendications 1 à 7, caractérisé en ce que le temps de contact, calculé dans les conditions réactionnelles, est compris entre 2 et 30 secondes, de préférence entre 5 et 25 secondes.

## Claims

1. Process for the manufacture of 1,1,1,2-tetrafluoroethane (F134a) by catalytic fluorination of 1-chloro-2,2,2 trifluoroethane (F133a) with the aid of anhydrous gaseous hydrofluoric acid, characterized in that a mixed catalyst is employed, composed of nickel and chromium oxides, halides and/or oxyhalides deposited on a support consisting of aluminium fluoride or of a mixture of aluminium fluoride and alumina.

2. Process according to Claim 1, characterized in that the weight content of nickel and chromium in the catalyst is between 0.5 and 20 % for each metal, the nickel/chromium atomic ratio being between 0.5 and 5, preferably close to 1.

3. Process according to Claim 2, characterized in that the content is between 2 and 10 % for each metal.

4. Process according to one of Claims 1 to 3, characterized in that the support may contain up to 1000 ppm of sodium.

5. Process according to one of Claims 1 to 4, characterized in that the reaction temperature is between 300 and 420°C, preferably between 330 and 410°C.

6. Process according to one of Claims 1 to 5, characterized in that the operating pressure is between 1 and 20 bars, preferably between 12 and 16 bars absolute.

7. Process according to one of Claims 1 to 6, characterized in that the HF/F133a molar ratio is between 1 and 20, preferably between 2 and 5.

8. Process according to one of Claims 1 to 7, characterized in that the contact time, calculated under the reaction conditions, is between 2 and 30 seconds, preferably between 5 and 25 seconds.

## Patentansprüche

1. Verfahren zur Herstellung von 1,1,1,2-Tetrafluorethan (F134a) durch die katalytische Fluorierung von 1-Chlor-2,2,2-trifluorethan (F133a) mit Hilfe von wasserfreier gasförmiger Fluorwasserstoffsäure, dadurch gekenn-zeichnet, daß ein Mischkatalysator verwendet wird, der aus Nickel- und Chromoxyden, -halogeniden und/oder -oxyhalogeniden besteht, die auf einem Trägermaterial aus Aluminiumfluorid oder aus einem Gemisch von Aluminium-fluorid und Aluminiumoxyd abgeschieden sind.

2. Verfahren gemäß Anspruch 1, dadurch gekennzeichnet, daß der Gewichtsanteil des Nickels oder des Chroms im Katalysator für jedes der Metalle zwischen 0,5 und 20 % beträgt, wobei das Atomverhältnis Nickel/Chrom zwischen 0,5 und 5, vorzugsweise nahezu 1 beträgt.

3. Verfahren gemäß Anspruch 2, dadurch gekennzeichnet, daß der Anteil für jedes der Metalle zwischen 2 und 10 % beträgt.

4. Verfahren gemäß einem der Ansprüche 1 bis 3, dadurch gekennzeichnet, daß das Trägermaterial bis zu 1000 ppm Natrium enthalten kann.

5. Verfahren gemäß einem der Ansprüche 1 bis 4, dadurch gekennzeichnet, daß die Reaktionstemperatur zwischen 300 und 420°C, vorzugsweise zwischen 330 und 410°C beträgt.

6. Verfahren gemäß einem der Ansprüche 1 bis 5, dadurch gekennzeichnet, daß der absolute Arbeitsdruck zwischen 1 und 20 bar, vorzugsweise zwischen 12 und 16 bar beträgt.

7. Verfahren gemäß einem der Ansprüche 1 bis 6, dadurch gekennzeichnet, daß das Molverhältnis HF/F133a zwischen 1 und 20, vorzugsweise zwischen 2 und 5 beträgt.

8. Verfahren nach einem der Ansprüche 1 bis 7, dadurch gekennzeichnet, daß die für die gegebenen Reaktionsbedingungen berechnete Kontaktzeit zwischen 2 und 30 Sekunden, vorzugsweise zwischen 5 und 25 Sekunden beträgt.
